# EUROPEAN PATENT APPLICATION

(11) **EP 4 487 878 A1**
(43) Date of publication of application: **08.01.2025**
(21) Application number: 22929252.9
(22) Date of filing: 01.03.2022
(51) Int. Cl.: A61L 27/36, A61L 27/38, A61L 27/52

(54) **WHARTON'S JELLY PRODUCT CAPABLE OF PROMOTING OSTEOGENESIS**

(71) Applicant: National Yang Ming Chiao Tung University, Taipei City 112304 (TW)
(72) Inventor: Fu, Yu-Show, Taipei (TW)
(74) Representative: Straus, Alexander
(86) International application number: PCT/CN2022/078588
(87) International publication number: WO 2023/164799

(57) **Abstract**

The present invention relates to a use of a medical product made from Wharton's jelly for promoting bone regeneration; wherein the medical product is obtained from one of the following two methods including the steps of: (1) taking a piece of Wharton's jelly from umbilical cords and treating it with a cryoprotective agent, and keeping it in freezing and thawing before use; and (2) taking a piece of Wharton's jelly from umbilical cords and freeze-drying it directly, and keeping it at a room temperature in dry conditions before use.

## Description

### FIELD OF THE INVENTION

The present invention pertains to a medical product made from Wharton's Jelly of umbilical cord with the effect of promoting bone regeneration.

### BACKGROUND OF THE INVENTION

Bones may be damaged or lost due to trauma, fracture, bacterial infection, or tumor metastasis and invasion. When bone loss is greater than two times the diameter of the diaphysis, known as critical-sized bone defect, the defect cannot naturally heal despite implanting various bone regenerative materials. Furthermore, the risks of reduced weight-bearing capability and subsequent fractures are increased. Therefore, how to repair of dense bone defects is a difficult problem in clinical orthopedics today.

Currently, common clinical bone materials are generally divided into three types. The first type is autologous bone graft whereby bone tissues from other body parts are surgically obtained and used to fill up the bone defect region. The drawbacks of this method include a long recovery period, the requirement for two invasive surgeries, and the amount/area of bone graft may not be large enough. The second type is allogeneic bone graft, i.e., excessive bone tissue removed from other patients who required surgical treatment. In addition to ethical issues, the risks of disease transmission are present. The third type is artificial bone materials, which require research and development of new synthetic bone materials for clinical applications.

### SUMMARY OF THE INVENTION

Therefore, the present invention provides a medical product made from Wharton's Jelly of umbilical cords, which has an effect of promoting bone regeneration, wherein the medical product is made by either of the two methods of the steps: (1) taking a piece of Wharton's Jelly from maternal umbilical cords, treating it with a cryoprotective agent and then freezing, and thawing before use; and (2) taking a piece of Wharton's Jelly from maternal umbilical cords and freeze-drying it directly, and keeping it at a room temperature in dry conditions before use.

According to the present invention, the medical product is obtained by the method (1), comprising the steps of taking a piece of Wharton's Jelly from maternal umbilical cords, treating it with a cryoprotective agent and then and then freezing, and keeping the product in a frozen status before use, which is named as WJF (Frozen Wharton's Jelly), retaining the cell bodies.

In an embodiment of the present invention, the cryoprotective agent is a solution containing 10% dimethyl sulfoxide (DMSO).

In an embodiment of the present invention, the frozen product is kept in liquid nitrogen.

In a particular example of the present invention, the medical product made from Wharton's Jelly is first protected with a cryoprotective agent (e.g., 10% DMSO solution) and then freezing it, and thawing before use.

According to the present invention, the medical product is obtained by the method (2), comprising the steps of taking a piece of Wharton's Jelly from maternal umbilical cords, freeze-drying it directly, and keeping it at a room temperature in dry conditions before use, which is named as WJD (Decellularize Wharton's Jelly matrix), containing no cell bodies.

In an embodiment of the present invention, the frozen products are stored at -80°C.

In a particular example of the present invention, the medical product is obtained by the method comprising taking a piece of fresh Warton's Jelly from maternal umbilical cords, freezing it directly at -80°C, and then freeze-drying it to drain the moisture.

According to the present invention, medical products made from Warton's Jelly obtained by the both methods can increase the performance of alkaline phosphatase in osteoblasts, increase the level of expression of bone differentiation genes in osteoblasts, promote the differentiation of osteoblasts, enhance the calcium deposition, effectively enhance the production of bone, enable the osteogenesis at a bone defect, enhance the activity of the osteoblasts within the bone defect, and induce the development of newly formed bone.

In one aspect, the present invention provides the use of the medical products made from Warton's Jelly in manufacturing a medicament for promoting bone regeneration.

It is to be understood that both the foregoing general description and the following detailed description are exemplary and explanatory only and are not restrictive of the invention.

### BRIEF DESCRIPTION OF THE SEVERAL VIEWS OF THE DRAWINGS

The foregoing summary, as well as the following detailed description of the invention, will be better understood when read in conjunction with the appended drawings. For the purpose of illustrating the invention, there are shown in the drawings embodiments which are presently preferred.

In the drawings:

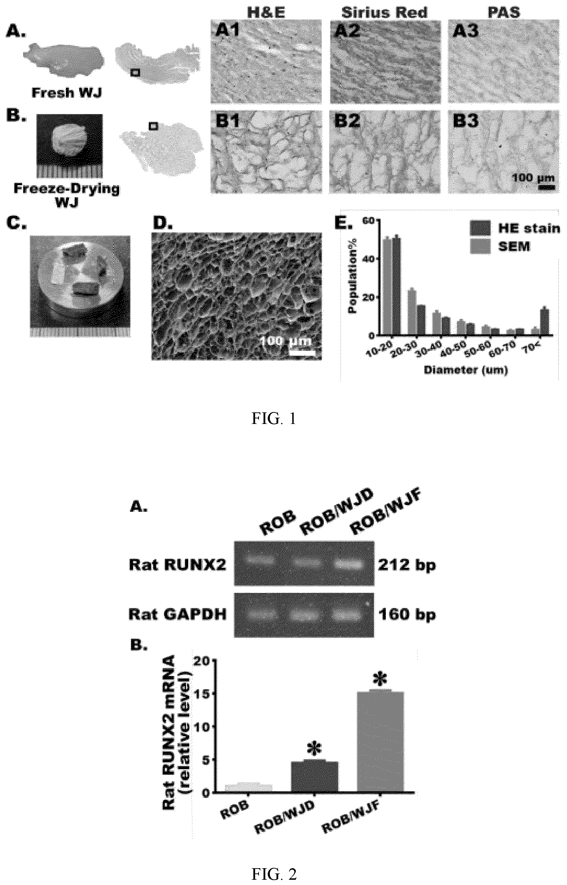

**FIG. 1****.** shows the histological and morphological analysis of freshly frozen, or decellularize Warton's Jelly of the present invention. In particular, FIG. 1A shows a freshly frozen Wharton's Jelly (WJF) removed from the umbilical cord, and FIG. 1B shows a decellularize Wharton's Jelly matrix (WJD), which was paraffin-embedded and sectioned. A1 and B1 are shown by H&E staining, the freshly WJF showed a dense structure and the tissue was rich in mesenchymal stem cells, while the WJD showed a sparse and porous structure. A2 and B2 are showed by Sirius Red stain, the collagen of freshly WJF is arranged in bundles and densely packed, whereas the collagen of WJD surrounds the periphery of the voids. A3 and B3 are showed by PAS stain, polysaccharides fill the tissue of the Warton's Jelly. FIG. 1C and FIG. 1D show the scanning electron microscopy of WJD showing porous structures of different sizes. FIG. 1E shows the freeze-dried Watton's Jelly, whether by paraffin-embedded sections, quantified by HE staining, or analyzed by scanning electron microscopy, showed porous structures of different sizes; moreover, the distribution of pore diameters was mostly in the range of 10-70 micrometers.
**FIG. 2****.** shows the co-culture of rat osteoblasts (ROB) with two Warton's Jelly enhances the expression of bone differentiation genes in osteoblasts. The co-culture of ROB with two Watten's Jelly enhanced the expression of osteogenic genes in osteoblasts. (A) ROB were cultured in vitro alone, with WJD, or with WJD. (A) ROB were cultured in vitro alone, with WJD, or with WJF for 14 days, and then subjected to RT-PCR for RUNX2. The RT-PCR assay for RUNX2 in rats was performed in all groups. (B) As well as the real time RT-PCR quantified the rat RUNX2 expression in ROB. The results showed a significant increase in the rat RUNX2 expression level in osteoblasts in the ROB/WJD group and the ROB/WJF group compared to the ROB group (p<0.05). *: Significant difference compared to ROB group, p<0.05.

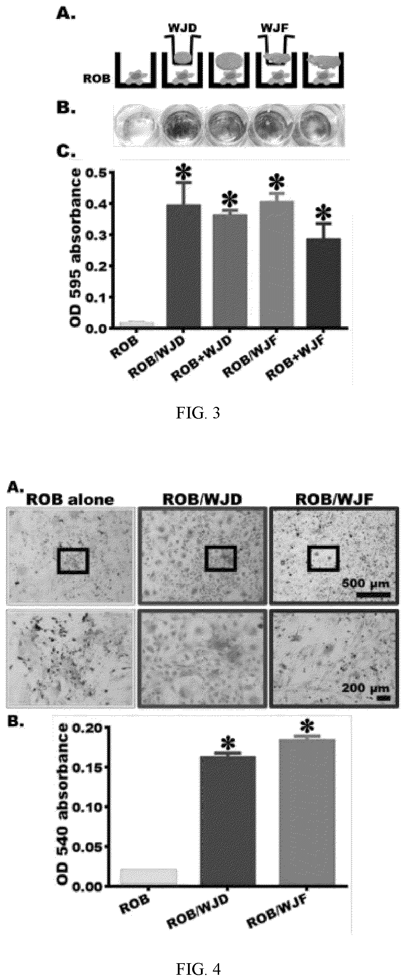

**FIG. 3****.** shows the quantification of alkaline phosphatase, co-cultured with WJD, or WJF, elevated the amount of alkaline phosphatase expression in ROB. Wherein: (A) is a graph of ROB, co-cultured non-directly or directly with WJD or WJF. (B) For cell staining for alkaline phosphatase after 14 days of culture. (C) For quantification of intracellular alkaline phosphatase concentration, the level of alkaline phosphatase expressed in ROB from the ROB/WJD group, the ROB+WJD group, the ROB/WJF group, or the ROB+WJF group was significantly increased compared to the ROB alone group as shown by OD 595 absorbance values (p<0.05). *: Significantly different compared to ROB group, p<0.05.
**FIG. 4****.** shows the performance of ROB co-cultured with two Warton's Jelly that promote osteoblast calcium deposition. (A) ROB cultured in vitro alone, co-cultured with WJD or WJF for 21 days, and stained with alizarin red S. The gradual magnification of the images showed that all groups of osteoblasts showed calcium deposition after treatment with bone differentiation medium. (B) To quantify the concentration of calcium ions in each group, the OD 540 absorbance values showed that the calcium deposition content in the ROB/WJD group and the ROB/WJF group was higher than that in the ROB group and the ROB+BMP2 group (p<0.05). *: Significant difference compared to ROB group, p<0.05.

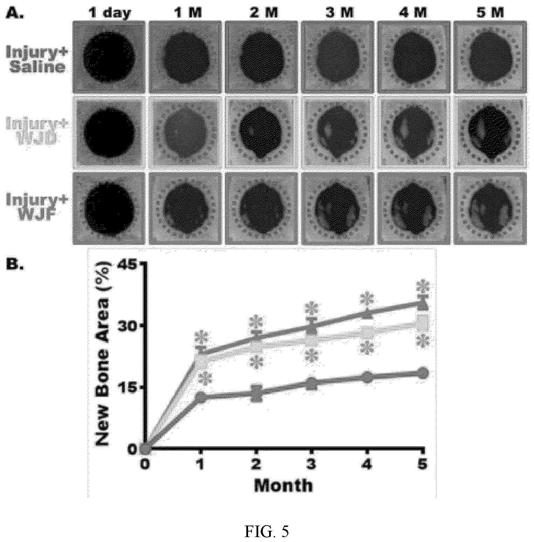

**FIG. 5****.** shows that the implantation of two Warton's Jelly in the treatment group effectively promoted osteogenesis in the calvarial defects of rats, as observed via micro-computed tomography (micro-CT). Bone healing in rats with cranial defects was tracked via micro-CT at the first, second, third, fourth, and fifth months after surgery. Therein: (A) In the Injury+Saline group, there was only a small amount of bone growth along the periphery of the defect, while in the Injury+WJD and Injury+WJF groups, there was free newly formed bone growth not only around the periphery of the defect, but also within the area of the defect. (B) Red dashed line marked the boundary of bone defect. Percentage of newly formed bone area was quantified in the boundary of bone defect. Results showed that the percentage of newly formed bone was significantly higher in the Injury+WJD and Injury+WJF groups compared to the Injury+Saline group *: Significantly different compared to the Injury+Saline group at the same month of age, p<0.05.

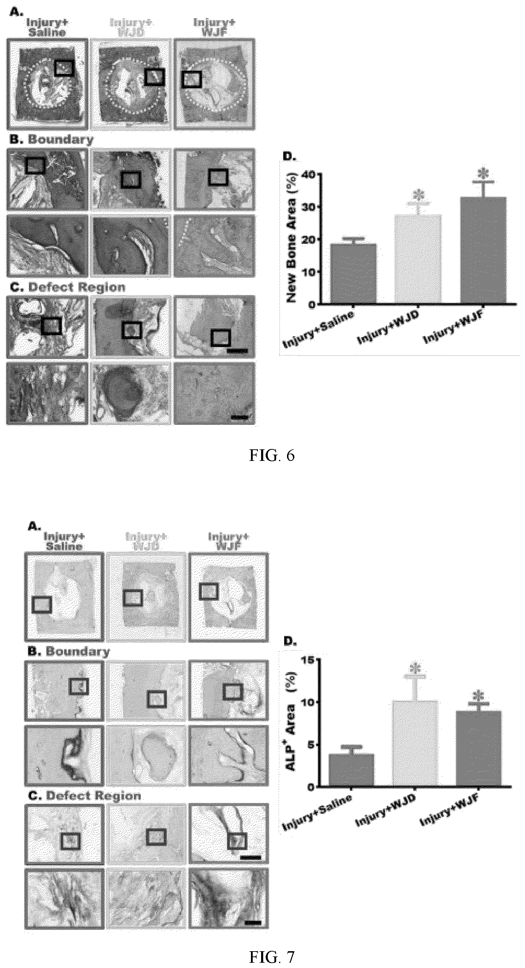

**FIG. 6****.** shows the percentage of area of newly formed bone effectively increased in calvarial defect rats by H&E staining observed in the treatment group implanted with two Warton's Jelly, respectively. (A) Low magnification panoramic views of skulls stained with ALP, and the area of the bone defect was marked by white dotted line, and then gradually enlarged the border areas of the bone defects from low magnification, respectively (B) or the inner area of the bone defect (C, scale bar is 1 mm and 200 µm, respectively) was gradually enlarged from low magnification. (D) Percentage of newly formed bone area was quantified by H&E staining, results showed that the percentage of distribution area of newly formed bone was significantly increased compared to the Injury+Saline group (p<0.05, n=5) in the Injury+WJD and Injury+WJF groups. *: Significant difference compared to Injury+Saline group, p<0.05.
**FIG. 7****.** shows the distribution of mature osteoblasts in rats with calvarial defects, as observed by ALP and implantation of two types of Walden's Jelly. (A) Low magnification panoramic views of skulls stained with ALP, and then gradually enlarged the border areas of the bone defects from low magnification, respectively (B), or the inner area of the bone defects (C), respectively. (D) Percentage of mature osteoblast area was quantified by ALP staining. Results showed that the percentage of distribution area of mature osteoblasts was significantly increased compared to the Injury+Saline group (p<0.05, n=5) in the Injury+WJD and Injury+WJF groups. *: Significant difference compared to Injury+Saline group, p<0.05.

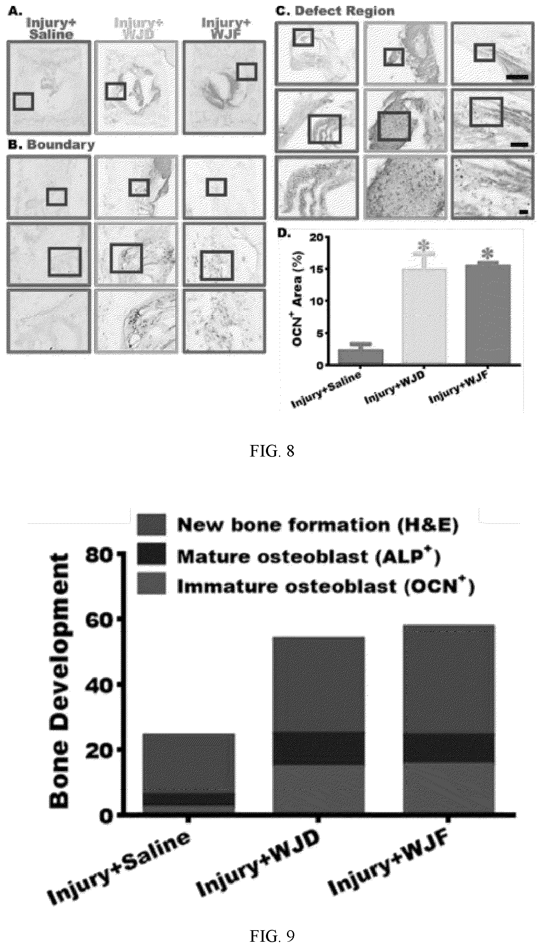

**FIG. 8****.** shows the distribution of immature osteoblasts in rats with cranial defects as observed by immunohistological staining with anti-osteocalcin and implantation of two Warton's Jelly. (A) Low magnification panoramic views of skulls stained with anti-osteocalcin, and then gradually enlarged the border areas of the bone defects from low magnification, respectively (B) or the inner area of the bone defects (C), respectively. (D) Percentage of immature osteoblast area was quantified by anti-osteocalcin staining. Results showed that the percentage of distribution area of immature osteoblasts was significantly increased compared to the Injury+Saline group (p<0.05, n=5) in the Injury+WJD and Injury+WJF groups. *: Significant difference compared to Injury+Saline group, p<0.05.
**FIG. 9****.** shows the trend of bone development in the newborn skull. Immunohistological staining with anti-osteocalcin was used to identify immature osteoblasts, ALP tissue staining to identify mature osteoblasts, and H&E staining to identify mature bone. The quantitative data from the three stains were summed to represent the different stages of bone development and to comprehensively assess the trend of osteogenesis in cranial defects in rats. Results showed that the development of newly formed bone was significantly higher when either freshly frozen, or decellularize Warton's jelly were implanted.

### DETAILED DESCRIPTION OF THE INVENTION

Unless defined otherwise, all technical and scientific terms used herein have the same meaning as commonly understood by a person skilled in the art to which this invention belongs.

As used herein, the singular forms "a", "an", and "the" include plural referents unless the context clearly dictates otherwise. Thus, for example, reference to "a sample" includes a plurality of such samples and equivalents thereto known to those skilled in the art.

The terms "including" or "comprising" are usually used in the sense of including/including allowing for the presence of one or more features, ingredients or components. The terms "including" or "comprising" cover the terms "consisting of" or "consisting of".

The terms "individual", or "subject" including a human and a non-human animal. For example, companion animal (dogs, cats, etc.), farms animal (cows, sheep, pigs, horses, etc.) laboratory animal (rats, and mice, guinea pig, etc.).

As used herein, the term Wharton's Jelly refers to the Wharton's Jelly in the umbilical cord of mammals, which is also known as inter-laminar jelly, namely, the mucus connective tissue material found in the umbilical cord that consists of a large amount of extracellular matrix that forms a colloid with a three-dimensional architecture, which is rich in extracellular matrix, collagen, glycoproteins, and hyaluronic acid. The umbilical cord contains two umbilical arteries and one umbilical vein; the connective tissue that surrounds the blood vessels is known as Wharton's Jelly.

A source suitable for preparing the product of the present invention is "Wharton's Jelly" from maternal umbilical cords, which may be processed, for example, by dissection, chopping, washing, enzymatic digestion, cryoprotection, freezing, drying, baking, or any combination thereof.

The term "critical-sized bone defect" as used herein refers to the destruction or removal of bone due to trauma, fracture, bacterial infection, or metastatic tumor. When the defect is more than twice the diameter of the long bone stem and does not heal naturally despite bone repair methods such as bone grafting, this phenomenon is called critical-sized bone defect.

The following examples are further provided to illustrate the present invention. According to the present disclosure, it should be comprehended by those skilled in the art that there may be changes or modifications therein, and still obtain similar or like results, but they do not depart from the spirit and scope of the present invention.

### Examples

### Material and methods

### 1.1 Preparation of Walden's Jelly

Human umbilical cords were aseptically collected after delivery and kept in cold (4°C) Hank's Balanced Salt Solution (HBSS; Gibco 14185-052). Wharton's jelly was isolated within 24 h after collection. All experimental instruments were autoclaved, disinfected in 75% ethanol, and flamed before using. In a laminar hood, umbilical cords were disinfected by soaking in 75% ethanol, placed in HBSS, and incised longitudinally. After the detachment and withdrawal of the blood vessels, we obtained Wharton's Jelly, which appeared as milky white interstitial tissues.

The isolated Wharton's jelly was subsequently processed using the following two methods:
(1). 1. Frozen Wharton's Jelly (WJF): Freshly obtained Wharton's jelly was immediately frozen in the presence of a cryoprotective agent. WJF was readily used after thawing, when it still contained HUMSCs.
(2). 2. Decellularized Wharton's Jelly matrix (WJD): Freshly obtained Wharton's jelly was stored in a -80°C refrigerator, then used a freeze dryer to drain the moisture in tissues, sealed and stored in a dry box for using.

WJF and WJD were used in the *in vitro* and animal experiments. In addition, WJF and WJD were paraffin-embedded and subjected to histochemical staining to examine their histological structures.

### 1.2 Tissue Fibers Staining

WJF and WJD were paraffin embedded. After deparaffinized and rehydrated, the tissue sections were stained using 0.1% Sirius red (Sigma 2610-10-8) in picric acid. Subsequently, the tissue sections were washed by immersion in ddH₂O. Tissue sections were then dehydrated by immersing in a series of increasing concentrations of alcohol, followed by immersing in xylene. Finally, the section slides were mounted with mounting medium and examined via an optical microscope.

### 1.3 Tissue Glycoproteins Staining

After WJF and WJD were deparaffinized and rehydrated, the tissue sections were stained with periodic acid-Schiff Stain (PAS stain) using the Periodic acid-Schiff Stain kit. The tissue sections were stained in periodic acid solution for 5 minutes, washed thrice in ddH₂O, 2 minutes for each time. And then, the tissue sections were stained in Schiff's reagent for 15 minutes, washed in ddH₂O for 5 minutes. After staining, the interstitial glycoproteins are pinkish-purple. Following dehydration with an increasing concentration alcohol and immersion in xylene, the sections were mounted with mounting medium and then examined via an optical microscope.

### 1.4 In vitro Culture of Rat Osteoblast (ROB)

One-day-old Sprague Dawley (SD) rats were sacrificed via anesthetic overdose, in a laminar flow cabinet, the rats' calvarias were obtained using sterilized instruments and cut into cubes with a side length of 1 mm, followed by washing with sterile HBSS to remove red blood cells. The tissues were then reacted with 0.1% trypsin and 0.1% collagenase for 2 hours at 37°C under shaking. Subsequently, Dulbecco's Modified Eagle Medium (DMEM) with 10% fetal bovine serum (FBS) was added to neutralize the effect of trypsin, followed by centrifugation at 1500 rpm for 5 minutes. The cells attached on the bottom were re-suspended in DMEM with 10% FBS. Cells were seeded in a 10-cm culture dish, and the culture medium was changed every 4 days. After 10 to 14 days, a full confluence could be reached, and cells were then passaged. Osteoblasts at the second passage were used in this study.

### 1.5 Grouping of in vitro Culture of Osteoblast

ROB cultured in vitro were divided into 3 groups as follows: (1) osteoblast group: 2×10⁴ ROB/well were cultured alone in a 24-well plate; (2) osteoblast/WJD group: 2×10⁴ ROB/well were seeded in the lower chamber of 24-well plate, and WJD was added to the top chamber of transwell; (3) osteoblast+WJD group: 2×10⁴ ROB/well were seeded in 24-well plate, and cultured with WJD; (4) osteoblast/WJF group: 2×10⁴ ROB/well were seeded in the lower chamber of 24-well plate, and WJF was added to the top chamber of transwell; (5) osteoblast+WJF group: 2×10⁴ ROB/well were seeded in 24-well plate, and cultured with WJF. Culture medium (DMEM with 10% FBS) was changed every 3 days. Alkaline Phosphatase Cell Stain were assessed after 14 days of culturing.

### 1.6 Reserve Transcription-Polymerase Chain Reaction (RT-PCR)

Cells were treated with 0.05% trypsin to be harvested and fresh calvarial tissue were then reacted with trizol (Sigma T9424) to extract total RNA by centrifugated at 12000g for 15 minutes at 4°C. Mixed the supernatant with equal volumes of isopropyl alcohol and centrifuged at 12000g for 10 minutes at 4°C. After washing the RNA with alcohol, redissolve it with DEPC water. Measure RNA concentration before use. The purified RNA was then transcribed to complementary DNA (cDNA) via reverse transcription (RT). The cDNA was subjected to polymerase chain reaction (PCR) with primers and master mix. The following primers were applied:
Rat-Runt-related transcription factor 2 (Rat-RUNX2) (212 bp)
   Forward: 5'-GCTTCTCCAACCCACGAATG -3'
   Reverse: 5' -GAACTGATAGGACGCTGACGA-3'
Rat-GAPDH (160 bp)
   Forward: 5'- CTCTACCCACGGCAAGTTCAAC-3'
   Reverse: 5'- GGTGAAGACGCCAGTAGACTCCA -3'

The PCR final products were analyzed using 2% agarose gel electrophoresis and photographed with a UV transilluminator.

### 1.7 Alkaline Phosphatase Cell Stain

Cells were washed by saline solution, cells were fixed using 2.5% glutardialdehyde for 15 minutes and then dissolved 2% 5-Bromo-4-Chloro-3-Indolyl Phosphate/Nitro Blue Tetrazolium (BCIP/NBT, Sigma B5655) in secondary water, and added to 24-well plate. For 1 hour in a 37°C oven in the dark. Subsequently, 10% cetylpyridinium chloride (Sigma C-0732) was added to HCl, for 1 hour in a 37°C oven in the dark. The absorbance of supernatant at 540 nm was measured to quantify each group of alkaline phosphatases. A higher absorbance indicates a higher level of alkaline phosphatase.

### 1.8 Alizarin red S Stain

ROB of osteoblast group, osteoblast/WJD group, and osteoblast/WJF group were cultured with osteogenic medium (R&D CCM007) for 21 days (the medium was changed every 3 days). Cells were fixed using 2.5% glutardialdehyde for 15 minutes and then stained with 2% alizarin red S (Sigma A5533) for 1 hour in a 37°C oven in the dark. Subsequently, 10% cetylpyridinium chloride (Sigma C-0732) was added to react for 1 hour in a 37°C oven in the dark. The absorbance of supernatant at 540 nm was measured and quantified the red area of each group. A higher absorbance indicates a higher level of calcium precipitation.

### 1.9 Experimental Animals

The experimental animals used in this experiment were used were eight-week-old male SD rats, weighed 250 to 280 g. The feeding method was a 45×24×20 cm transparent PC cage, 12 hours of light per day (7:30 am to 7:30 pm), ambient temperature constant temperature air conditioning (22 ±2 °C) and adequate supply of feed and drinking water, change litter once a week.

### 1.10 Establishment of critical-sized cranial bone defect model in rats

SD rats were anesthetized via intraperitoneal injection of Zoletil 50 and xylazine hydrochloride (Sigma 23076359), the rat's head was fixed in a stereotaxic apparatus and the hair was shaved with a razor. Used a razor to shave the hair cleanly, the rat's skin was then disinfected with iodine solution-dipped cotton ball and incised with a scalpel, the subcutaneous tissues were pushed away using a surgical scissor, and the periosteum was removed. Subsequently, a surgical trephine bur (8 mm in diameter) was applied to drill the calvaria, and the calvaria was detached from the underlying dura to serve as a critical-sized bone defect animal model. Finally, the subcutaneous tissues and skin were sutured.

### 1.11 Grouping of Experimental Animals

SD rats were grouped into 3 study groups:
1. Injury+Saline group: no treatment but saline was given following calvarial bone defect.
2. Injury+WJD group: Dissolved WJD in saline was administered to the defected region immediately after calvarial bone defect.
3. Injury+WJF group: Dissolved WJF in saline was administered to the defected region immediately after calvarial bone defect.

### 1.12 micro-Computerized Tomography Scan (micro-CT)

Micro-CT images were processed with AMIDE software (SOURCEFORGE) to reconstruct 3-dimentional radiographic images. The Small Animal Computed Tomography System (U-CT^{UHR}, MILabs) was applied in the present study to scan live rats' calvarias on Day 1 after surgery and monthly at Months 1, 2, 3, 4, and 5 to follow-up the healing levels of calvarial bone defect.

### 1.13 Sacrifice, Perfusion Fixation, Decalcification, and Paraffin Sections of Experimental Animals

SD rats were anesthetized via intraperitoneal injection of Zoletil 50 and xylazine hydrochloride (Sigma 23076359), and fixed via perfusion of 4% paraformaldehyde and 7.5% picric acid in 0.01M PBS. The calvarias were obtained and placed in fixative solution for 24 hours. For decalcification, calvarias were then immersed in 20% EDTA in 9% NaOH solution for 9 days, with the solution changed twice. The decalcified calvarias were washed in running water for 8 hours, followed by dehydration with ethanol solution and xylene, and paraffin-embedded with pure paraffin. Subsequently, the samples were horizontally sectioned into 7-µm thick tissue slices with a microtome.

### 1.14 Hematoxylin & Eosin Stain

Tissue sections were deparaffinized, rehydrated, stained with hematoxylin (Muto Pure Chemicals; No. 3008-1) for 5 minutes, then washed in running water for 30 minutes. After placing 70% alcohol for 30 minutes, placed the sections in a ventilated environment for the remaining alcohol to evaporate. Subsequently, tissue sections were stained with eosin (Muto Pure Chemicals; No. 3008-2) for 1 minute, then washed in running water for 15 minutes, and the sections were dehydrated with an increasing concentration ethanol, and treated with xylene twice. Finally, the slides were mounted with mounting medium for examination and photography via an optical microscope. The slice with the largest area of calvarial tissue was obtained, and the circumference of the boundaries between the old and newly formed bone were marked for quantification of the original area of bone defect. Subsequently, the newly formed bone within the bone defect region was quantified and expressed in percentage.

### 1.15 Alkaline Phosphate Stain (ALP Stain)

Calvarial tissue sections were deparaffinized, rehydrated, and reacted with Alkaline Phosphatase kit (Sigma 85L2-1KT) for 1 hour in a 37°C oven, followed by immersion in ddH₂O. The slides were mounted with mounting medium for examination, photography and quantification via an optical microscope. Stained sections could be observed a cell stained in purple indicated that the sections contained ALP, i.e., the cell was a mature osteoblast. The slice with the largest area of calvarial tissue was obtained, and the circumference of the boundaries between the old and newly formed bone were marked for quantification of the original area of bone defect. Subsequently, the newly formed bone within the bone defect region was quantified and expressed in percentage as an area proportion of the osteoblasts.

### 1.16 Anti-osteocalcin antibody Immunohistochemistry Staining

Following deparaffinization of cranial tissue sections, primary mouse anti-osteocalcin antibody (Abcam ab13740, 1:200) was added to react for 16 to 18 hours at 4°C. Subsequently, secondary goat anti-mouse-IgG-conjugated biotin (Millipore AP124B, 1:250) was added to react for 1 hour at room temperature, followed by avidin-biotinylated-horseradish peroxidase complex (ABC Kit, Vector Laboratories) (Vector Laboratoris PK-4000, VECTASTAIN^{®}) for 1 hour at room temperature. The sections were then washed thrice with 0.01M PBS for 5 minutes. Finally, DAB (5 mg DAB, 35% H₂O₂ 3.5 µL in 10 mL Tris-HCl, pH 7.4) was added for color development. After then, air dried the sections at room temperature. The sections were dehydrated with an increasing concentration ethanol, and treated with xylene. Finally, the slides were mounted with mounting medium for examination and photography via an optical microscope.

### 1.17 Statistical Analysis

All experimental data were expressed in mean ± standard deviation. One-way ANOVA was applied for comparing means of each study groups, and two-way ANOVA was applied, followed by Fisher's least significant difference (LSD) test for multiple comparisons. A value of p < 0.05 was considered a lowest standard for statistical significance.

### 2. Results

### 2.1 Histological and Morphological Analysis of WJD and WJF

WJD of an umbilicus cord (FIG. 1A) and WJF (FIG. 1B) were paraffin-embedded for serial tissue sectioning. With H&E staining, observed the cellular and tissue structure of Wharton's Jelly. Results showed that there were a large number of nuclei in WJF, the structure was also dense and neatly arranged (FIG. 1A1). After lyophilized Wharton's Jelly, there are no nuclei could be observed, the structure was turned from dense to loose, and showed the holes of all sizes. The components of Wharton's Jelly were the collagens and glycoproteins, and stained with Sirius red and PAS respectively, observed the distribution of the collagens and glycoproteins. Results showed that the collagens and glycoproteins in Wharton's Jelly were not be destroyed by lyophilization, and still serving as a supporting structure for Wharton's Jelly (FIG. 1A2-A3, B2-B3). Observed the cell morphology via a scanning electron microscope (FIG. 1C). Scanning images showed that WJD profile revealed densely distributed pores (FIG. 1D). Randomly selected multiple views, and measured the hole size, calculated the range distribution of average hole diameter. Analysis results showed that WJD, whether paraffin-embedded for sectioning and quantified by H&E staining, or analyzed via a scanning electron microscope, revealed the multiple holes structure of various sizes; and the range distribution of hole diameter was in the range of 10 to 70 microns in WJD (FIG. 1E).

### 2.2 Co-culture of rat Osteoblasts with Warton's Jelly enhances the expression of Bone Differentiation Genes in Osteoblasts

In vitro culture of rat osteoblast (ROB) individually, or co-cultured with WJD or WJF for 14 days, after treated with trypsin for RNA extraction. RT-PCR was using rat runt-related transcription factor 2 (RUNX2) to discuss the bone differentiation of ROB. Results showed that GAPDH was detected in the ROB group, ROB/WJD group, or ROB/WJF group; and RUNX2 was also detected in all groups (FIG. 2A). Further, differences in the expression level of gene between groups were analyzed using a real-time quantitative polymerase chain reaction apparatus. Quantitative results showed that the expression level of rat RUNX2 on osteoblast in ROB/WJD group, or ROB/WJF group was significantly increased compared with the ROB group (FIG. 2B).

### 2.3 Co-culture of rat Osteoblasts with Warton's Jelly enhances the expression of Alkaline Phosphatase in Osteoblasts

ROB were cultured in vitro individually, or co-cultured non-directly, or directly in vitro with WJD and WJF, respectively. Treated with DMEM with 10% FBS for 14 days, and then the cells were stained with BICP/NBT, and absorbance were measured by OD595 to observe the expression level of alkaline phosphatase in ROB (FIG. 3A, FIG. 3B). Results showed that the alkaline phosphatase expression level in ROB was observed by measuring the absorbance value with OD595 (FIG. 3A, FIG. 3B). The results showed that the intracellular expression of alkaline phosphatase in ROB co-cultured with WJD or WJF was significantly increased compared with that of osteoblasts cultured individually (p<0.05, FIG. 3C).

### 2.4 Co-culture of rat Osteoblasts with Warton's Jelly increases the Level of Calcium precipitation in Osteoblasts

ROB were cultured in vitro individually, or co-cultured non-directly, or directly in vitro with WJD and WJF, respectively. Treated with bone differentiation medium for 21 days, and then the cells were stained with alizarin red S, and marked the manifestation of calcium deposition in ROB in dark red color. Gradually enlarge the display from a low-magnification image, there were clusters of osteoblasts that were sparsely grouped in the ROB group, and there was only a low level of calcium deposition in the field of view. In addition to clusters of osteoblasts, concentric circles of osteoblasts in the ROB/WJD group, and there was a high level of calcium deposition in the surrounding area. Mature osteoblasts with tubules could be found in the ROB/WJF group, and there were clusters of osteoblasts surrounded by them. There are also a large number of calcium crystals distributed under the field of view (FIG. 4A). Subsequently, the calcium crystals marked in dark red were dissolved, and the concentration of calcium ions was analyzed by measuring the absorbance with OD540. Results showed that the concentration of calcium ions in the ROB/WJD group, or the ROB AVJF group, was significantly increased compared with the ROB group (p<0.05, FIG. 4B). It was hypothesized from the results of alizarin red S staining that non-directly co-culturing with WJD, or WJF could promote the differentiation of osteoblasts and enhance the manifestation of calcium deposition.

### 2.5 Quantification of Bone Regeneration in Critical-sized Calvarial Defect via micro-Computed Tomography Scan shows that Warton's Jelly effectively increase the Bone Production

Calvarial bone healing was observed via micro-computed tomography (micro-CT) at the first, second, third, fourth, and fifth months after calvarial defect surgery in rats. The newly formed bone grew inwardly along the border of the bone defect in a small amount in the Injury+Saline group. In addition to the inwardly growing newly formed bone, also showed sporadic newly formed bone in the defect area in the Injury+WJD group or Injury+WJF group (FIG. 5A). The percentage of area of newly formed bone was quantified in the area of the calvarial defect. Results showed that the Injury+Saline group had 12.5±3.4 % of newly formed bone in the first postoperative month, and the calvarial healed very slow as the increase of postoperative time, the calvarial bone recovered in the fifth postoperative month with only 18.4±1.4 %. In the Injury+WJD and Injury+WJF groups, the percentage of newly formed bone was 21.2+3.9% and 23.0:1:4.1 %, respectively, in the first month after surgery, which significantly increased (p<0.05) compared with the Injury+Saline group, and the increasing was maintained until the fifth month, with the percentage of newly formed bone in the calvarial defect reaching 30.6±.6 %, and 35.6±3.7 %, respectively (p<0.05, FIG. 5B). Observation via micro-CT macroscopy, the calvarial bone healing capacity of rats operated for transcranial bone defects was only maintained in the range of 12% to 18% without any treatment over a period of five months. WJD or WJF could promote osteogenesis in rats with calvarial bone defects.

### 2.6 H&E staining shows that Warton's Jelly promote the Bone Regeneration in Critical-sized Calvarial Defect of rats

A low magnification panorama of the H&E staining results (FIG. 6A) was used to gradually magnify the bony performance from low magnification at the border of defect (FIG. 6B), and the central area of bone defect (FIG. 6C). Results showed that there was a small amount of newly formed bone and dense connective tissue at the border of the defect in the Injury+Saline group; and there was only connective tissue within the bone defect. In addition to a tendency to increase the amount of bone at the border of the bone defects, free and maturing newly formed bone was also observed within the bone defect in the Injury+WJD and Injury+WJF groups. The area percentage of newly formed bone within the bone defect was quantified by H&E staining at the fifth month after surgery. Only 18.8±1.9 % of newly formed bone within the bone defect in Injury+Saline group. In contrast, the area percentage of the newly formed bone within the bone defect reached 27.1±4.0 % and 32.7±5.0 % in the Injury+WJD and Injury+WJF groups, respectively, which was a significant increase compared with the Injury+Saline group (p<0.05, FIG. 6D). As microscopically observed by H&E staining, WJD or WJF promoted bone neogenesis in rats with calvarial defects.

### 2.7 Osteoblast marking via Alkaline Phosphate Staining shows that Warton's Jelly increase the Active Performance in Critical-sized Calvarial Defect of rats

A low magnification panorama of the ALP staining results (FIG. 7A) were showed to gradually magnify the area of mature osteoblast performance from low magnification at the border of defect (FIG. 7B), and the central area of bone defect (FIG. 7C). Results showed that a small number of mature osteoblasts in the Injury+Saline group showed ALP at the border and the central of the bone defect. The mature osteoblasts adhered to inwardly growing newly formed bone at the border of the bone defect in both the Injury+WJD and Injury+WJF groups, while free newly formed bone was surrounded by osteoblasts within the bone defect. The area percentage of ALP⁺ in the defect was quantified by ALP staining. ALP⁺ area was only about 3.7±2.1 % in the Injury+Saline group. ALP⁺ area reached 10.0±5.9 % and 8.8±2.3 % in the Injury+WJD and Injury+WJF groups, respectively, which was a trend increase compared with the Injury+Saline group (p<0.05, FIG. 7D). As microscopically observed by ALP staining, WJD or WJF increased the activity of osteoblasts in rats with calvarial defects.

### 2.8 Newly Formed Bone marking via Anti-osteocalcin Antibody Immunohistochemistry Staining shows that Warton's Jelly promote the Performance of Bone Regeneration in Critical-sized Calvarial Defect of rats

Osteocalcin is secreted by osteoblasts and released into bone tissue to form uncalcified bone during osteogenesis. A low magnification panorama of anti-osteocalcin immunostaining (FIG. 8A) results was showed to gradually magnify the expression level of osteocalcin-containing uncalcified bone at the border of the bone defect (FIG. 8B) and in the area within the bone defect (FIG. 8C). Results showed that in the Injury + Saline group there were no osteocalcin been observed at the border of the bone defect; only a small amount of osteocalcin was released into the tissues within the bone defect in the Injury + Saline group. In the Injury+WJD and Injury+WJF individual groups, osteocalcin was not only at the border of the bone defect or in the bone defect, but also in clusters of osteoblasts, which mean osteoblasts undergoing an osteogenic reaction. The percentage of OCN⁺ area within the bone defect was quantified by OCN staining. Only 2.4±1.6 % of OCN⁺ area in the Injury+Saline group. In contrast, the percentage of OCN+ area reached 14.8±4.8 %, and 15.5±1.5 %, respectively in the Injury+WJD, and Injury+WJF individual groups, which was a significant increase compared with the Injury+Saline group (p<0.05, FIG. 8D). As microscopically observed by anti-osteocalcin staining, WJD or WJF further promoted the performance of osteoblasts to promote bone regeneration.

### 2.9 Warton's Jelly could obviously promote the Development of Newly Formed Bone

Immature osteoblasts were marked with anti-osteocalcin immunostaining, mature osteoblasts were marked with ALP tissue staining, and mature bone were marked with H&E staining, respectively. Quantitative data from the three staining were summed to assess bone development in calvarial defects of rats. Results showed that the development of bone was approximately 25% in the Injury+Saline group, while approximately 52% and 57% in the Injury+WJD and Injury+WJF groups, respectively, which was higher development of bone (FIG. 9).

### 3. Conclusion

Warton's Jelly were transplanted into rats with calvarial defects. It was found that after treatment with either WJF or WJD, the performance of newly formed bone was observed by micro-CT, and staining of the sections showed that the signals presented in the images were mature bone, which also promoted the development of the host bone. According to the above experimental results, Warton's Jelly can be used as a natural medical material to promote bone regeneration in clinical medicine, so that patients who still suffering from bone defects can be provided with new treatment methods.

## Claims

1. A medical product for promoting bone regeneration, wherein the medical product is prepared by two methods including the following steps: (1) taking a piece of Warton's Jelly from maternal umbilical cords, treating it with a cryoprotective agent and then freezing, and thawing before use; and (2) taking a piece of Warton's Jelly from maternal umbilical cords, directly freeze-drying it, and keeping it at a room temperature in dry conditions before use.

2. The medical product of claim 1, wherein the umbilical cords are from human or non-human mammals.

3. The medical product of claim 1, wherein the cryoprotective agent is 10% Dimethyl sulfoxide (DMSO) solution.

4. The medical product of claim 1, wherein the step of freezing performs in liquid nitrogen.

5. The medical product of claim 1, 2, 3 or 4, which contains cell bodies.

6. The medical product of claim 1, wherein the step of freeze-drying comprises taking a piece of fresh Warton's Jelly from maternal umbilical cords, freezing it directly at -80°C, and freeze-drying to drain the moisture.

7. The medical product of claim 6, which contains no cell bodies.

8. The medical product of claim 1, which increases the performance of alkaline phosphatase in osteoblasts, increases the level of expression of bone differentiation genes in osteoblasts, promotes the differentiation of osteoblasts, enhances the calcium deposition, effectively enhance the production of bone, enables the osteogenesis within the bone defect, enhances the activity of the osteoblasts within the bone defect, or induces the development of newly formed bone.

9. A use of the medical product of claim 1 for manufacturing a medicament for promoting bone regeneration.

10. A use of claim 9, wherein the medical product increases the performance of alkaline phosphatase in osteoblasts, increases the level of expression of bone differentiation genes in osteoblasts, promotes the differentiation of osteoblasts, enhances the calcium deposition, effectively enhances the production of bone, enables the osteogenesis within the bone defect, enhance the activity of the osteoblasts within the bone defect, or induces the development of newly formed bone.
